(19) Europäisches Patentamt
European Patent Office
Office européen des brevets

(11) EP 0 995 450 B1

(12) EUROPÄISCHE PATENTSCHRIFT

(45) Veröffentlichungstag und Bekanntmachung des Hinweises auf die Patenterteilung:
**26.06.2002 Patentblatt 2002/26**

(51) Int Cl.$^7$: **A61L 27/16**

(21) Anmeldenummer: **99810845.0**

(22) Anmeldetag: **22.09.1999**

# (54) UHMW-Polyethylen für Implantate

UHMW-polyethylene for implants

Polyéthylène à ultra haut poids moléculaire (UHMW-PE) pour implants

(84) Benannte Vertragsstaaten:
**AT BE CH CY DE DK ES FI FR GB GR IE IT LI LU MC NL PT SE**

(30) Priorität: **21.10.1998 EP 98811048**

(43) Veröffentlichungstag der Anmeldung:
**26.04.2000 Patentblatt 2000/17**

(73) Patentinhaber: **Sulzer Orthopädie AG**
**6340 Baar (CH)**

(72) Erfinder: **Schaffner, Silvio**
**8267 Berlingen (CH)**

(74) Vertreter: **Manitz, Finsterwald & Partner Gbr**
**Postfach 31 02 20**
**80102 München (DE)**

(56) Entgegenhaltungen:
**EP-A- 0 542 108**
**EP-A- 0 613 923**
**CS-B- 221 403**
**US-A- 5 650 485**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 995 450 B1

## Beschreibung

Die Erfindung handelt von der Herstellung von UHMW-Polyethylen für Implantate.

Nur wenige Firmen haben sich auf die Herstellung von UHMW-Polyethylen für medizinische Zwecke spezialisiert. Eine übliche Herstellung benutzt Pulver oder Granulat, welches bei Temperaturen um 180°C - 240°C und Drükken um 2 - 10 MPa zu Fertigteilen oder zu Blöcken verpresst wird oder zu Stangen extrudiert wird, aus denen Implantatteile, beispielsweise die Lagerschalen von künstlichen Hüftgelenken oder künstlichen Kniegelenken gefertigt werden.

Eine übliche Weiterverarbeitung der Implantatteile besteht darin, die Teile in einer Schutzgasatmosphäre beispielsweise in Stickstoff in einem Beutel einzuschweissen und in diesem Beutel durch Bestrahlung mittels $\gamma$-Strahlen oder Elektronenstrahlen zu sterilisieren. In diesem Zustand sind die Implantatteile lagerfähig und stehen jederzeit für eine Implantation zu Verfügung.

Die Bestrahlung mittels $\gamma$-Strahlen oder Elektronenstrahlen bringt neben der Sterilisation den Effekt, dass im Polyethylen auch Kettenstrukturen mit freien Radikalen entstehen, welche zu einer Vernetzung und/oder zu einer Oxidation führen wenn Sauerstoffatome verfügbar sind.

Sauerstoff ist zum einen im Polyethylen vorhanden und kann zum anderen langsam in das Polyethylen hineindiffundieren, was dazu führt, dass nach einigen Jahren eine Versprödung des Materials einsetzt, welche die mechanischen Eigenschaften reduziert und zu einem erhöhten Verschleiss führt.

In der Patentanmeldung EP-A-0 613 923 (HOECHST AG) wird ein Verfahren zur Herstellung einer stabilisierten Formmasse aus UHMW-Polyethylen von $10^5$ bis $10^7$ g/mol beschrieben, welche Antioxidantien und insbesondere $\alpha$-Tocopherol enthält, um Implantate langlebiger zu machen. Zur Herstellung werden die Inhaltsstoffe gemischt, bei Temperaturen von 180° bis 250°C und Drucken von 3 bis 5 MPa gesintert und bei Drucken von 7 bis 10 MPa abgekühlt.

In der Schrift CS 221403 vom 15.09.1982 wird über eine nicht gesundheitsschädliche Stabilisierung von Polyolefinen gegen Thermooxidation und gegen Photooxidation berichtet. Verschiedene Polyolefine altern langsamer unter natürlichen Umweltbedingungen, wenn man ihnen während der Herstellung ein Tocopherol beimischt. Im Gegensatz zu anderen Antioxidanzien wie Derivaten von Phenolaminen und Phosphiden, die gesundheitlich bedenklich sein können, wird vorgeschlagen Polyolefinen, die in der Lebensmittelindustrie, im Gesundheitswesen oder als Implantate verwendet werden, Tocopherol zuzumischen, um eine langsamere Alterung zu erreichen. Dabei sollen Gewichtsanteile von 0,01 % bis 5 % an Tocopherol bei den Polyolefinen die Thermooxidation bei der Herstellung des Materials und die Photooxidation des Materials unter natürlichen Umweltbedingungen verlangsamen.

In der Patentschrift US 5,650,485 (HOWMEDICA INC.) wird die Herstellung von UHMW-Polyethylen beschrieben. Dabei werden praktisch alle Verfahrensschritte bis zum gebrauchsfertigen Implantat in Abwesenheit von Sauerstoff durchgeführt, damit dieser nicht in das Material eindiffundiert und die Vernetzung von freien Radikalen bei nachfolgender $\gamma$-Bestrahlung mit 2,5 Mrad verhindert.

Hier setzt die Erfindung ein. Sie hat die Aufgabe möglichst gleichmässig die Oxidation von UHMW-Polyethylenimplantaten zu verhindern.

Diese Aufgabe wird mit dem im unabhängigen Anspruch 1 gezeigten Verfahren gelöst.

Es hat sich nämlich gezeigt, dass dispers verteiltes Vitamin E die freien Radikalen schneller als eindiffundierender Sauerstoff absättigen kann und somit ein Fortschreiten der Oxidation verhindert. Es hat sich ebenfalls gezeigt, dass ein zu grosser Prozentsatz an eingelagerten Vitamin E beispielsweise über 1 % ebenfalls zu einer Verschlechterung der mechanischen Eigenschaften führt, indem sich der E-Modul, die Zugfestigkeit und die Kerbschlagzähigkeit des Materials verschlechtern. Andererseits kann die Oxidation bereits bei einem Anteil an Vitamin E der grösser als 0,01 % ist, spürbar beeinträchtigt werden. Es kommt eben darauf an, die disperse Verteilung so gleichmässig wie möglich und mit einem Aufwand, der sich wirtschaftlich noch rechtfertigen lässt, vorzunehmen.

Die abhängigen Patentansprüche 2 bis 6 entsprechen Weiterbildungen des Verfahrens. Je besser es gelingt gleichmässig kleine Teilchen zu erzeugen und gleichmässig an ihrer Oberfläche mit Vitamin E zu dotieren, desto enger und wirkungsvoller sind die erreichbaren Grenzen für eine mittlere Konzentration von Vitamin E, die in einer verbesserten Näherung $0{,}1\ \% < \bar{K} < 0{,}4\ \%$ und bei verbesserter Auslese und Benetzung der PE-Teilchen $0{,}1\ \% < \bar{K} < 0{,}2\ \%$ betragen kann.

Sehr kleine Teilchen aus UHMW-Polyethylen haben eine relativ grosse Oberfläche im Verhältnis zu ihrem Volumen. Wenn es gelingt diese Teilchen sehr fein zu mahlen, was mit Ultraschallmühlen, die mit sich kreuzenden Schallfronten kleinste Teilchengrössen erzeugen können, möglich ist, dann kann Vitamin E, welches in einer Flüssigkeit als Suspension gelagert ist oder welches in einem Lösungsmittel enthalten ist, in einer kleinen und beherrschbaren Konzentration mit der Flüssigkeit an den Polyethylenteilchen angelagert werden. Je ähnlicher die Polyethylenteilchen im Durchmesser sind desto ähnlicher ist auch ihr Faktor für das Verhältnis von Oberfläche zu Volumen und die Gewissheit bei einer bestimmten Oberflächenspannung der Flüssigkeit gleiche Mengen von Vitamin E an den Teilchen anzulagern, wenn die Flüssigkeit verdampft wird, was auch bei niederen Temperaturen im Vakuum möglich ist. Als

Lösungsmittel für Vitamin E sind zum Beispiel Alkohole geeignet. Ein so entstandenes Feinstpulver welches gleichmässig mit einer niedrigen Konzentration von Vitamin E dotiert ist, lässt sich sofort oder nach Zwischenlagerung in einer neutralen Atmosphäre zu den erwähnten Bedingungen in kompakte Blöcke und Tafeln verpressen. Das Vitamin E ist dispers eingelagert und erfüllt seine Funktion erst, wenn fertig bearbeitete Implantatteile zur Sterilisation und Bildung von freien Radikalen mit einer γ-Strahlung von mindestens 2,5 Mrad bestrahlt worden sind. Die nach der Bestrahlung noch vorhandenen freien Radikale werden schneller vom Vitamin E als vom Sauerstoff abgesättigt und verhindern so eine frühzeitige Oxidation und Alterung. Natürliches Vitamin E enthält neben dem Hauptbestandteil α-Tocopherol noch β-Tocopherol und weitere Isomere, die eine etwas geringere Wirkung als Antioxidans haben.

α-Tocopherol mit der Strukturformel

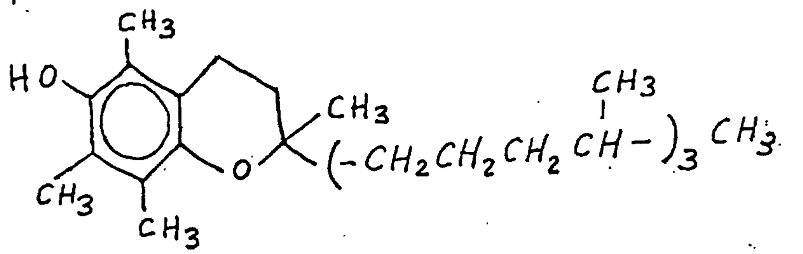

entspricht einem handelsüblichen Vitamin E in Form einer pasteusen Masse, die lösbar ist in organischen Lösemitteln wie Alkohole, Ketone und flüssigen Alkanen z.B. Ethanol, Aceton oder n-Heptan. Bei der Festlegung der Lösungskonzentration und -menge muss die Oberfläche der Polyethylenteilchen und die des Mischgefässes berücksichtigt werden, um die PE-Teilchen gleichmässig und in einer ihrem Volumen entsprechenden Konzentration K von Vitamin E mit Lösungsmittel und Vitamin E zu benetzen.

Das Verdampfen vom Lösungsmittel kann in einem Autoklav durch Aufrechterhalten eines Vakuums vorgenommen werden.

Um nicht gewollten Reaktionen des Vitamins E vorzubeugen, welches auf eine grosse Oberfläche verteilt ist, kann man die mit Vitamin E belegten Polyethylenteilchen im Vakuum oder in einer Schutzgasatmosphäre lagern.

Das Implantat wird durch spanende Formgebung aus den Blöcken oder Stangen weiterverarbeitet und innerhalb einer Schutzgasatmosphäre eingeschlossen. Zur γ-Sterilisation und Vernetzung durch freie Radikale wird es einer Strahlungsmenge von mindestens 2,5 Mrad ausgesetzt.

Einzelne Verfahrensschritte können in einer Schutzgasatmosphäre ohne Sauerstoff vorgenommen werden.

## Patentansprüche

1. Verfahren zum Herstellen eines Implantates aus UHMW-Polyethylen, wobei

    - in einem ersten Schritt Pulver aus UHMW-Polyethylenteilchen mit einer Flüssigkeit gemischt wird, die Vitamin E in einer vorgegebenen Menge enthält,
    - in einem zweiten Schritt die Flüssigkeit verdampft wird und das Vitamin E in einer vorgegebenen Konzentration $0{,}1\ \% < \bar{K} < 0{,}4\ \%$ auf den Polyethylenteilchen angelagert wird,
    - in einem dritten Schritt die Polyethylenteilchen bei Temperaturen um 180°C - 240°C und Drücken um 2- 10 MPa zu Blöcken verpresst oder zu Stangen extrudiert werden.
    - in einem vierten Schritt aus den Blöcken oder Stangen das Implantat in seiner endgültigen Form durch spanende Formgebung herausgeschnitten wird,
    - in einem fünften Schritt das Implantat in einer Schutzgasatmosphäre in einer Umhüllung eingeschlossen wird und während einer γ-Sterilisation einer Strahlung von mindestens 2,5 Mrad ausgesetzt wird, damit freie Radikale gebildet werden.

2. Verfahren nach Anspruch 1, **dadurch gekennzeichnet, dass** die Flüssigkeit ein Lösungsmittel für Vitamin E ist, beispielsweise ein Alkohol.

3. Verfahren nach Anspruch 1, **dadurch gekennzeichnet, dass** die Flüssigkeit eine Trägerflüssigkeit ist, in der das Vitamin E als Suspension eingelagert ist.

4. Verfahren nach Anspruch 1, **dadurch gekennzeichnet, dass** einzelne Schritte in einer Schutzgasatmosphäre ohne Sauerstoff ausgeführt werden.

5. Verfahren nach einem der Ansprüche 1 bis 4, **dadurch gekennzeichnet, dass** als Vitamin E ein α-Tocopherol mit einer Strukturformel

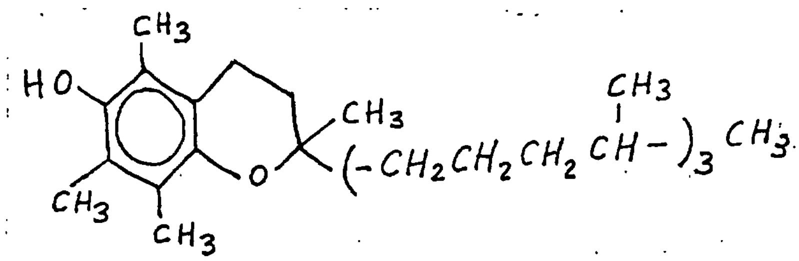

verwendet wird.

6. Verfahren nach Anspruch 1, **dadurch gekennzeichnet, dass** in einer Vorstufe Polyethylenteilchen von annähernd gleicher Grösse aus dem Pulver separiert werden, um bei einer bestimmten Oberflächenspannung der Flüssigkeit gleiche Mengen von Vitamin E an diesen Teilchen anzulagern und anschliessend die Flüssigkeit zu verdampfen.

## Claims

1. Method for the manufacture of an implant of UHMW polyethylene, wherein

- in a first step a powder of UHMW polyethylene particles is mixed with a liquid which contains vitamin E in a predetermined amount,

- in a second step the liquid is evaporated and the vitamin E is deposited in a predetermined concentration of 0.1 % < $\bar{k}$ < 0.2 % on the polyethylene particles,

- in a third step the polyethylene particles are compressed to blocks or extruded to rods at temperatures around 180°C - 240°C and pressures around 2 - 10 MPa,

- in a fourth step the implant is cut out of the blocks or rods into its final form by chip-forming shaping,

- in a fifth step the implant is enclosed in a cover in a protective gas atmosphere and is exposed to a radiation of at least 2.5 Mrad during a γ-sterilisation so that free radicals are formed.

2. Method in accordance with claim 1, **characterised in that in that** the liquid is a solvent for vitamin E, for example an alcohol.

3. Method in accordance with claim 1, **characterised in that** the liquid is a carrier liquid in which the vitamin E is stored as a suspension.

4. Method in accordance with claim 1, **characterised in that** individual steps are carried out in a protective gas atmosphere without oxygen.

5. Method in accordance with one of the claims 1 to 4, **characterised in that** an α-tocopherol with a structure formula

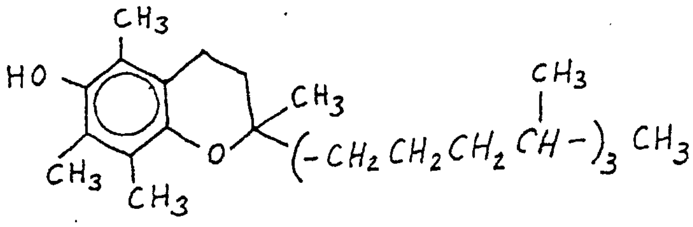

is used as the vitamin E.

6. Method in accordance with claim 1, **characterised in that** polyethylene particles of approximately the same size are separated out of the powder in a pre-stage in order to deposit like quantities of vitamin E on these particles for a specific surface tension of the liquid and subsequently to vaporise the liquid.

## Revendications

1. Procédé de fabrication d'un implant en polyéthylène UHMW, où

   - lors d'une première étape, de la poudre de particules de polyéthylène UHMW est mélangée avec un liquide qui contient de la vitamine E en une quantité prédéterminée,
   - lors d'une deuxième étape, le liquide est évaporé, et la vitamine E est déposée en une concentration prédéterminée de 0,1% < $\bar{k}$ < 0,4% sur les particules de polyéthylène,
   - lors d'une troisième étape, les particules de polyéthylène sont comprimées en blocs ou sont extrudées en tiges à des températures autour de 180°C - 240°C et des pressions autour de 2 - 10 MPa,
   - lors d'une quatrième étape, l'implant est découpé des blocs ou tiges dans sa forme définitive par une configuration à enlèvement des copeaux,
   - lors d'une cinquième étape, l'implant est enfermé dans une atmosphère de gaz de protection dans une enveloppe et est exposé pendant une stérilisation $\gamma$ à un rayonnement d'au moins 2,5 Mrad pour que des radicaux. libres soient formés.

2. Procédé selon la revendication 1, **caractérisé en ce que** le liquide est un solvant pour la vitamine E, par exemple un alcool.

3. Procédé selon la revendication 1, **caractérisé en ce que** le liquide est un liquide porteur dans lequel la vitamine E est incluse comme suspension.

4. Procédé selon la revendication 1, **caractérisé en ce que** des étapes individuelles sont effectuées dans une atmosphère de gaz de protection sans oxygène.

5. Procédé selon l'une des revendications 1 à 4, **caractérisé en ce qu'**on utilise comme vitamine E un $\alpha$-tocophérol d'une formule de structure

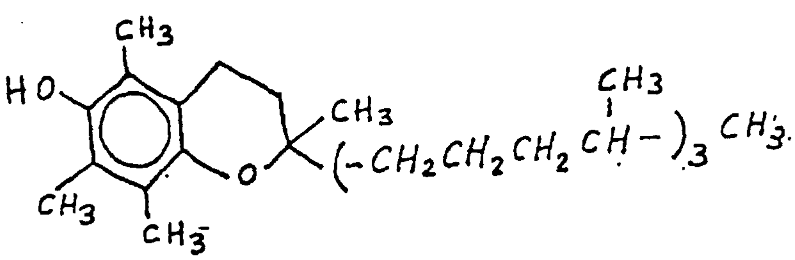

6. Procédé selon la revendication 1, **caractérisé en ce que** lors d'une étape préalable, des particules de polyéthylène approximativement de la même grandeur sont séparées de la poudre pour, lors d'une tension de surface déterminée du liquide, déposer des quantités égales de vitamine E sur ces particules et faire évaporer ensuite le liquide.